# EUROPEAN PATENT APPLICATION

(11) **EP 4 389 086 A1**
(43) Date of publication of application: **26.06.2024**
(21) Application number: 22215694.5
(22) Date of filing: 21.12.2022
(51) Int. Cl.: A61F 5/01, A61H 1/02, B25J 9/00, B25J 9/10

(54) **TENDON-DRIVEN WEARABLE ORTHOSIS AND METHOD FOR CALIBRATING A TENDON-DRIVEN WEARABLE ORTHOSIS**

(71) Applicant: Universität Heidelberg, 69117 Heidelberg (DE); Morgalla, Maximilian, 69214 Eppelheim (DE)
(72) Inventor: MORGALLA, Maximilian, 69214 Eppelheim (DE); ALICEA, Ryan, 69115 Heidelberg (DE); MASIA, Lorenzo, 69121 Heidelberg (DE)
(74) Representative: Müller-Boré & Partner Patentanwälte PartG mbB

(57) **Abstract**

The disclosure concerns a tendon-driven wearable orthosis configured to be worn by a user, wherein the tendon-driven wearable orthosis is configured to assist a movement of at least one joint of the user, comprising: an adapter unit connectable, via at least one tendon, to at least one end effector, wherein the adapter unit is releasably connectable to a motor unit, wherein the motor unit is configured to operate the adapter unit to exert a pulling force on the at least one tendon to assist the movement of the at least one joint, wherein the adapter unit comprises at least one tensioning device configured to adjustably control a length of the at least one tendon between the adapter unit and the at least one end effector. Furthermore, a method for calibrating a tendon-driven wearable orthosis configured to be worn by a user is disclosed.

## Description

The present invention relates to a tendon-driven wearable orthosis and a method for calibrating a tendon-driven orthosis.

The invention lies in the field of user wearable orthoses, and in particular in the field of tendon-driven wearable orthoses. Specifically, members of a population suffering from some form of, for example, upper body paralysis from a stroke, spinal cord injury, brachial plexus injury, or other causes may struggle to perform certain movements, such as grasping movements normally executed by a hand. To aid in performing such movements or restoring the ability to perform said movements, different types of mechanical devices have previously been developed, such as powered glove devices to be worn on the hand of a user. The powered glove devices may be actuated using tendon-based and pneumatic systems.

Typically available tendon-based systems comprise an actuator connected to a glove structure via one or more tendons. Each tendon is of a fixed length and recovered and released by the actuator. It is therefore necessary to perform accurate measurements of the user to ensure accurate values for tendon lengths are determined. Subsequently, the tendons are cut to size and fixedly connected to the actuator and the glove structure. Failure to obtain accurate measurements may cause incorrect operation of the tendon-based system. For example, when an underactuated tendon-based system is used, inaccurate measurements may cause one or more fingers to flex/extend out of synchronisation, leading to an unnatural finger movement and/or failure to safely grasp objects.

In addition, the specific required lengths for each tendon are highly user-specific, such that a mass production of tendon-based systems is not possible. This may be avoided by providing separate tendons and corresponding separate actuators for each tendon. However, such an approach significantly increases power requirements, as well as system complexity, costs, mass, and size for the orthosis.

It is therefore an object of the invention, to provide a tendon-driven wearable orthosis with improved individualisation properties. The above object is solved by a tendon-driven wearable orthosis and a method of calibrating a tendon-driven wearable orthosis according to the independent claims. Preferred embodiments form the subject of the dependent claims.

An aspect of the disclosure relates to a tendon-driven wearable orthosis configured to be worn by a user. In particular, the tendon-driven wearable orthosis may be at least partially mountable on a harness, wherein the tendon-driven wearable orthosis and/or the harness may be configured to be worn by the user. The harness may comprise, for example, one or more belts and/or one or more bands. The harness may comprise at least one of one or more textiles, one or more plastics, one or more metals, and/or one or more composite materials. The harness may be a unitary harness or may comprise one or more distinct harness parts, such as for example belts and/or bands.

The tendon-driven wearable orthosis is configured to assist a movement of at least one joint of the user. A joint of a user may connect two or more structures, such as for example bones, of a skeletal system of the user. The tendon-driven wearable orthosis may be configured to assist the movement of the at least one joint by generating a direct and/or indirect force, such as a pulling force, on at least one structure to cause a movement, such as a bending movement, of the at least one joint of the user. The tendon-driven wearable orthosis may be configured to assist a movement of multiple joints of the user simultaneously, such as by assisting a bending motion of a finger and/or hand of the user.

The tendon-driven wearable orthosis comprises an adapter unit connectable, via at least one tendon, to at least one end effector. In particular, at least one tendon may be fixedly or releasably connectable to the adapter unit at a first end of the at least one tendon, and fixedly or releasably connectable to at least one end effector at a second end of the at least one tendon. Alternatively or additionally, at least one tendon may be configured as at least one closed loop tendon, wherein the at least one closed loop tendon may be fixedly or releasably connectable to the adapter unit and at least one end effector.

The at least one tendon may be at least one cable element, such as a metal cable. However, the tendon-driven wearable orthosis is not limited to such a type of tendon, and any type of tendon may be used. All of the at least one tendon may be formed from a substantially identical material, or different materials may be used. Furthermore, the term "tendon" is intended to be interpreted broadly herein, and may thus also encompass other elements suitable to connect the adapter unit to the at least one end effector. For example, the at least one tendon may alternatively or additionally comprise one or more of a band, a strap, a chain, a string, and/or a ribbon. Furthermore, the at least one tendon may be configured as at least one inner cable of at least one Bowden cable.

The at least one end effector may be configured to be worn by the user, such as for example on a hand and/or finger of a user. However, the tendon-driven wearable orthosis is not limited thereto, and may be used for other elements and/or joints of the user. The at least one end effector may be configured to be fixedly or releasably connectable to the at least one tendon. In particular, the at least one end effector may comprise one or more connection elements configured to be connectable to the at least one tendon, wherein the one or more connection elements may comprise one or more hooks, loops, and/or tendon guides. The at least one end effector may, for example, be configured to be mountable on a support structure, such as a glove structure, configured to be worn by the user. The tendon-driven wearable orthosis may further comprise at least one tendon guide element configured to guide the at least one tendon from the adapter unit to the at least one end effector and/or the support structure. The at least one tendon guide element may comprise a Bowden cable outer sheath, wherein each of the at least one tendon may be configured as inner cables positioned inside the Bowden cable outer sheath. The at least one tendon guide element may be fixedly connected, at a first end thereof, to the adapter unit, and fixedly connected, at a second end thereof, to the support structure. Specifically, it may therefore be possible to maintain a desired distance between the support structure and the adapter unit, while still allowing the length of the at least one tendon to be adjustably controlled to, for example, pre-tension said at least one tendon.

The adapter unit is releasably connectable to a motor unit. For example, the adapter unit may be mountable on and/or engageable with the motor unit. The motor unit is configured to operate the adapter unit to exert a pulling force on the at least one tendon to assist the movement of the at least one joint. In other words, the motor unit is configured to operate the adapter unit to generate the pulling force, wherein the pulling force is exertable on the at least one tendon to assist the movement of the at least one joint. For example, in a state in which the adapter unit is connected to the motor unit, the motor unit may be configured to generate a force, such as a mechanical force, on the adapter unit to cause the adapter unit to exert the pulling force. The pulling force exerted on the at least one tendon may be configured to cause, via the at least one tendon, a further pulling force to be exerted on the at least one end effector to assist the movement of the at least one joint. The motor unit may, for example, be configured as a linear actuator or a rotary actuator. The motor unit may be configured as a non-backdriveable motor unit. In other words, the motor unit may be configured to operate the adapter unit in one operating direction (e.g., forward or clockwise) instead of two operating directions (e.g., forward and backward, or clockwise and counter-clockwise). Specifically, a very simple design of the motor unit may thus become possible.

The adapter unit further comprises at least one tensioning device configured to adjustably control a length of the at least one tendon between the adapter unit and the at least one end effector. The at least one tensioning device may be configured to adjustably control the length of the at least one tendon to pre-tension the at least one tendon between the adapter unit and the at least one end effector. Adjustably controlling the length of the at least one tendon may in particular be understood as adjustably increasing and/or decreasing the length of the at least one tendon. It may therefore be possible to adjustably control the length of the at least one tendon to have a desired length.

Thereby the adapter unit may advantageously allow an efficient individualisation of the tendon-driven wearable orthosis to specific needs or parameters of the user, such as for each finger of the user separately. In particular, it may thus be possible to avoid having to perform significant measurements of the user even prior to producing the tendon-driven wearable orthosis in order to ensure an individual fit to the user. Furthermore, by providing such a tendon-driven wearable orthosis comprising the adapter unit, it may become possible to provide said tendon-driven wearable orthosis as a standardised product, which can efficiently and easily be adapted to the individual needs of the user. Specifically, mass production of the tendon-driven wearable orthosis may thus be achieved.

In addition, by providing a tendon-driven wearable orthosis comprising the adapter unit, the tendon-driven wearable orthosis may be dynamically adjusted to the user. Therefore, errors in measurements of the user can be prevented from causing a wrong fit and/or operation of the orthosis. In addition, the tendon-driven wearable orthosis may be adapted to potential physiological changes of the user.

The use of underactuation of the tendon-driven wearable orthosis may also be efficiently enabled. For example, the adapter unit and/or two or more tendons may be configured to be operable by a single actuator of a motor unit. Thereby, requirements of the motor unit may be significantly reduced (such as, for example, power, cost, complexity, mass, and size requirements), while still maintaining large degrees of freedom for any user. Flexibility of the tendon-driven wearable orthosis may further be improved by allowing the adapter unit to be efficiently attached to and removed from the motor unit.

Furthermore, by providing such a tendon-driven wearable orthosis, the tendon-driven wearable orthosis can be worn by one or more users, while allowing an individual fit to be obtained for each of said users as desired and/or required by a current wearer of the tendon-driven wearable orthosis.

The tendon-driven wearable orthosis may be configured to be at least partially worn on a hand and/or arm of the user. Specifically, the adapter unit may be connectable, via the at least one tendon, to the at least one end effector, wherein said at least one end effector may be configured to be worn and/or mounted on a hand and/or at least one finger of the user. Thereby, the adapter unit may be configured to exert the pulling force, via the at least one tendon and the at least one end effector, on at least one joint of the hand of the user. In particular, the tendon-driven wearable orthosis may be configured to assist a movement of at least one finger joint of a hand of the user. The movement of the at least one finger joint may be a flexion and/or extension of the at least one finger joint.

Therefore, the tendon-driven wearable orthosis may efficiently and easily assist the movement of the hand of the user, and may consequently support such a movement for users suffering from mobility issues of their hand or hands, due to for example a stroke.

In particular, the tendon-driven wearable orthosis may be configured to assist a substantially simultaneous movement of at least two finger joints of at least two of the index finger, the middle finger, the ring finger, the little finger, and/or the thumb. Specifically, by assisting such a substantially simultaneous movement, it may be possible to support grasping motions of the hand of the user very efficiently.

"Substantially" is to be understood, in the context of the present disclosure, as also comprising small deviations caused by, for example, environmental and/or construction/production reasons, unless a different definition is specifically provided.

The tendon-driven wearable orthosis may further comprise the at least one tendon and the at least one end effector. In particular, the tendon-driven wearable orthosis may comprise the adapter unit, the at least one tendon, and the at least one end effector, wherein the adapter unit is connected, via the at least one tendon, to the at least one end effector. It may therefore be possible to efficiently provide the tendon-driven wearable orthosis as a unit configured to be individually adjustable to the requirements of the user via, for example, the at least one tensioning device.

The tendon-driven wearable orthosis may further also comprise the motor unit.

The at least one tensioning device may be configured to adjustably control the length of the at least one tendon between the adapter unit and the at least one end effector while the adapter unit is not connected to the motor unit. In particular, it may therefore be possible to individually adjust the tendon-driven wearable orthosis to the user without requiring a connection to the motor unit. Therefore, an easier and simpler construction of both the motor unit and the adapter unit may be achieved. In addition, the process of adjusting the length of the at least one tendon may be further facilitated, as the adapter unit may be in a state separate from the motor unit.

The adapter unit may further comprise at least one first spool, wherein the at least one tendon may be connectable to the at least one first spool. In particular, the adapter unit may comprise one first spool for each tendon of the at least one tendon. Each tendon may be connectable to one respective first spool. Thereby, an efficient management of the at least one tendon in the tendon-driven wearable orthosis may be achieved. The at least one first spool is configured to be rotatable by the motor unit to exert the pulling force on the at least one tendon. In particular, the at least one first spool may be configured to be, in a state in which the adapter unit is connected to the motor unit, operatable by the motor unit to rotate around a corresponding rotation axis. Each first spool may have its own rotation axis, or one or more spools may share a common rotation axis. The at least one first spool may be configured such that the at least one tendon may be at least partially wound around the at least one first spool by rotation of the at least one first spool.

The at least one first spool may further comprise one or more spool bearings, such as one or more spool bearing washers. It is noted that while the provision of one or more spool bearings is described for the at least one first spool herein, it is to be understood that any spool described herein may further comprise one or more spool bearings.

The at least one tensioning device may comprise at least one first tensioning tool releasably engageable with the adapter unit and the at least one first spool. Releasably engageable is to be understood as encompassing being configured to be brought into engagement and being configured to be brought out of engagement, as desired, preferentially without causing substantial damages and/or deformations. Furthermore, the at least one first spool may be configured to be operable, preferentially rotatable, using the at least one first tensioning tool to control the length of the at least one tendon between the adapter unit and the at least one end effector. Preferentially, the at least one first spool may be configured to be rotatable using the at least one first tensioning tool to reduce the length of the at least one tendon between the adapter unit and the at least one end effector. Specifically, it may therefore be possible to very efficiently control the length of the at least one tendon, enabling an improved calibration of the tendon-driven wearable orthosis to the individual requirements of the user.

The at least one first tensioning tool may be at least one ratcheting key. The at least one ratcheting key may be configured to be operable to perform a ratcheting operation. In particular, each ratcheting key may comprise a plurality of first ratcheting teeth and the adapter unit may comprise a plurality of second ratcheting teeth.

Ratcheting teeth may be understood within this disclosure as comprising a series of, preferentially alternating, protrusions and recesses. The series of protrusions and recesses may respectively extend radially outward or inward with respect to a respective central axis of an element or structure comprising said series of protrusions and recesses. Ratcheting teeth, as described herein may be configured to be at least partially elastically deformable.

Each ratcheting key may be configured to be releasably engageable with the adapter unit and at least one first spool to bring the plurality of first ratcheting teeth into engagement with at least a subset of the plurality of second ratcheting teeth. The plurality of first ratcheting teeth and at least the subset of plurality of second ratcheting teeth may be configured to prevent rotation of the at least one first spool in a first rotation direction and allow rotation of the at least one first spool in a second rotation direction opposite to the first rotation direction, while the plurality of first ratcheting teeth is in engagement with at least the subset of plurality of second ratcheting teeth. In particular, the first rotation direction and the second rotation direction may correspond to opposite rotation directions around a central rotation axis of the at least one ratcheting key and/or the at least one first spool. Specifically, it may therefore be efficiently possible to reduce the length of the at least one tendon and maintaining such a reduced length of the at least one tendon using the at least one ratcheting key. This may allow the length of multiple tendons to be adjusted in sequence, without having to actively maintain any such tendons at the reduced and/or desired length, such as by holding the respective ratcheting key manually by the user. Furthermore, should a length of a tendon have accidentally been reduced too much and/or tensioned too tightly, the respective ratcheting key may simply be brought out of engagement with the adapter unit and the at least one first spool to allow the at least one first spool to rotate to correct said length.

The at least one tensioning device may further comprise a carriage movably mounted on a carriage track, wherein the carriage may be a spool carriage. The carriage or spool carriage may for example comprise a carriage body and at least one wheel element, wherein the at least one wheel element is fixed to the carriage body and configured to allow the carriage or spool carriage to be movable along the carriage track. The carriage track may comprise one or more guide rails, along which the carriage or spool carriage may be guided.

The at least one tensioning device may further comprise at least one connection device mounted on the carriage, wherein the at least one tendon may be connectable to the at least one connection device, wherein the carriage may be configured to be movable along the carriage track by the motor unit to exert the pulling force on the at least one tendon.

The at least one connection device may be implemented as at least one second spool, as further described below. However, the at least one connection device is not limited to such an implementation, and any at least one connection device configured to be connectable to the at least one tendon could be implemented. For example, the at least one connection device may be implemented as at least one adjustable clamp. Preferably such a clamp is configured to be operated with one hand, e.g. without a winding pulley, which enable to reduce the overall size of the connection. For example, a thumb screw can be tightened onto a clamping plate which holds the tendons in place at the correct relative lengths. Such operation might be performed by a technicial during the initial fitting of the product, for example.

The at least one tensioning device may further comprise at least one second spool mounted on the spool carriage, wherein the at least one tendon is connectable to the at least one second spool. The at least one second spool may be mounted on the spool carriage to be rotatable around a central rotation axis of the at least one second spool. Furthermore, the at least one second spool may be configured such that the at least one tendon may be at least partially wound around the at least one second spool by rotation of the at least one second spool. The spool carriage may further be configured to be movable along the carriage track by the motor unit to exert the pulling force on the at least one tendon.

In particular, it may therefore be possible to efficiently cause the pulling force to be exerted using, for example, a linear actuator configured to move the carriage or spool carriage along the carriage track.

The spool carriage may further comprise a plurality of third ratcheting teeth. In particular, the plurality of third ratcheting teeth may be provided in the spool carriage body of the spool carriage. The at least one second spool may comprise a plurality of fourth ratcheting teeth releasably engageable with at least a subset of the plurality of third ratcheting teeth. The plurality of fourth ratcheting teeth and at least the subset of plurality of third ratcheting teeth may be configured to prevent rotation of the at least one second spool in a third rotation direction and allow rotation of the at least one second spool in a fourth rotation direction opposite to the third rotation direction, while the plurality of fourth ratcheting teeth is in engagement with at least the subset of plurality of third ratcheting teeth. In particular, the third rotation direction and the fourth rotation direction may correspond to opposite rotation directions around a central rotation axis of the at least one second spool. Specifically, it may therefore be efficiently possible to reduce the length of the at least one tendon and maintaining such a reduced length of the at least one tendon.

The at least one tensioning device may further comprise at least one second tensioning tool releasably engageable with the at least one second spool. In particular, the at least one second spool may be configured to be rotatable in the fourth direction using the at least one second tensioning tool to control the length of the at least one tendon between the adapter unit and the at least one end effector. For example, the at least one second tensioning tool may be configured to be engageable with at least one second spool and further comprise a rotation handle configured to allow the user to rotate the at least one second tensioning tool to cause a rotation of the at least one second spool.

The at least one second spool may be further configured to be operable using the at least one second tensioning tool to move the plurality of fourth ratcheting teeth out of engagement with at least the subset of the plurality of third ratcheting teeth. In particular, the at least one second spool may comprise at least one spring element. For example, the at least one second spool may be at least one second spring-loaded spool. The at least one spring element may be fixed at a first spring end to the spool carriage and/or the carriage body, and/or may be fixed at a second spring end to the at least one second spool. A compression direction of the at least one spring element may be substantially parallel to a central rotation axis of the at least one second spool. The at least one second tensioning tool may be operable to cause a pressing force on the at least one second spool to cause the at least one spring element to be compressed, thereby moving the plurality of fourth ratcheting teeth out of engagement with at least the subset of the plurality of third ratcheting teeth.

Thereby, a particularly efficient and easy to use means to adjustably control the length of the at least one tendon may be provided. A length of the at least one tendon may easily be reduced by engaging the at least one second tensioning tool with the at least one second spool and rotating said at least one second tensioning tool. In addition, should a length of a tendon have accidentally been reduced too much and/or tensioned too tightly, a pressing force may be exerted on the respective second tensioning tool, such as by the user, in order to cause the plurality of fourth ratcheting teeth to be brought out of engagement with at least the subset of the third ratcheting teeth. Thereby, the at least one second spool may be allowed to rotate in the third rotation direction to increase the length of the respective at least one tendon.

The at least one tensioning device may further comprise a multi-spool unit. The multi-spool unit may comprise at least a central rotation shaft and a plurality of third spools. Each of the third spools may be substantially identical to one another. The plurality of third spools may be mounted on the central rotation shaft. For example, each third spool may comprise a central mounting bore extending through the respective third spool, wherein the central rotation shaft is insertable into the central mounting bore. The at least one tendon may be connectable to the at least one third spool, wherein the at least one tendon may be configured to be wound around the at least one third spool during rotation of the at least one third spool. In particular, using such a multi-spool unit, it may be possible to efficiently and simultaneously exert the pulling force on the at least one tendon by rotation of the central rotation shaft.

Furthermore, the multi-spool unit may be configured to be switchable between a first state and a second state. In the first state, each of the plurality of third spools may be configured to be rotatable, preferentially freely rotatable, around the central rotation shaft. In the second state, the plurality of third spools may be configured to be fixed relative to the central rotation shaft. For example, in the second state, the plurality of third spools may be compressed between a top plate and a bottom plate of the multi-spool unit, wherein the top plate and the bottom plate may be engageable to and/or fixed relative to the central rotation shaft. Furthermore, in the second state, the multi-spool unit may be configured to be rotatable by the motor unit to exert the pulling force on the at least one tendon. Thereby a simple design and operation of the multi-spool unit may be achieved.

The at least one tensioning device may further comprise at least one third tensioning tool. The at least one third tensioning tool may be configured to be releasably engageable with the plurality of third spools in the first state to fix an orientation of the plurality of third spools relative to one another. For example, each of the plurality of third spools may comprise at least one locking bore extending through the respective third spool. The at least one third tensioning tool may be insertable into at least one locking bore of each third spool in order to fix the orientation of the plurality of third spools with respect to one another. In particular, it is therefore possible to rotate each of the third spools in the first state around the central rotation shaft into a desired respective orientation and then insert the at least one third tensioning tool into the respective at least one locking bores. Specifically, it is therefore possible to individually select a length for each tendon connected to a respective third spool. The at least one third tensioning tool may for example be at least one locking screw, wherein the at least one locking screw may be configured to be screwed into the top plate and/or the bottom plate. The at least one third tensioning tool may be removable from the multi-spool unit in the second state. Thereby, a simple and efficient means of adapting the multi-spool unit and thus the tendon-driven wearable orthosis to individual requirements of the user may be provided.

For example, each of the plurality of third spools may comprise eight locking bores extending through the respective third spool. However, the plurality of third spools are not restricted to such a number of locking bores, wherein the number of locking bores may be freely chosen according to the requirements of the tendon-driven wearable orthosis and/or the user. Specifically, by increasing the number of locking bores, more desired respective orientations and/or a higher precision of the multi-spool unit may be achievable. Furthermore, by reducing the number of locking bores, a structural stability of the multi-spool unit may be increased, in light of for example the materials used for the multi-spool unit, and therefore a failure chance of the multi-spool unit may be reduced. Therefore, the number of locking bores may be chosen by, for example, weighing such factors against one another and finding an acceptable compromise according to the requirements of the tendon-driven wearable orthosis and/or the user.

The adapter unit may further comprise a tensioning device for each one of the at least one tendon, wherein each tensioning device is configured to adjustably control the length of a respective tendon between the adapter unit and the at least one end effector. Alternatively, the adapter unit may comprise a tensioning device for a plurality of tendons or all of the tendons, wherein each tensioning device is configured to adjustably control the length of the respective tendons between the adapter unit and the at least one end effector.

The motor unit may be configured to operate the adapter unit to exert the pulling force on the at least two tendons substantially simultaneously to assist the movement of the at least one joint. Preferentially, the motor unit may be configured to operate the adapter unit to exert the pulling force on all tendons substantially simultaneously to assist the movement of the at least one joint, thereby efficiently allowing the tendon-driven wearable orthosis to assist, for example, a grasping motion of the user.

The adapter unit may be configured to be connectable to the motor unit and the at least one first tensioning tool, the at least one second tensioning tool, and/or the at least one third tensioning tool, respectively, at the same time. It may therefore be possible to maintain a desired length of the at least one tendon using the at least one first tensioning tool, the at least one second tensioning tool, and/or the at least one third tensioning tool, respectively, until the adapter unit is connected to the motor unit. Specifically, it may therefore be possible to avoid unwanted and/or unintentional changes in the length of one or more tendons during connection of the adapter unit with the motor unit.

A further aspect of the disclosure concerns a method for calibrating a tendon-driven wearable orthosis configured to be worn by a user, wherein the tendon-driven wearable orthosis is configured to assist a movement of at least one joint of the user. The tendon-driven wearable orthosis comprises an adapter unit connectable, via at least one tendon, to at least one end effector. The method comprises mounting the tendon-driven wearable orthosis on the user, and adjustably controlling, using at least one tensioning device of the adapter unit, a length of the at least one tendon between the adapter unit and the at least one end effector.

The step of adjustably controlling the length of the at least one tendon may further comprise releasably connecting at least one tensioning tool to the adapter unit. The at least one tensioning tool may, for example, be at least one first tensioning tool, at least one second tensioning tool, and/or at least one third tensioning tool, as described herein.

The step of adjustably controlling the length of the at least one tendon may further comprise operating, such as for example rotating and/or turning, using the at least one tensioning tool, the at least one tensioning device to control the length of the at least one tendon between the adapter unit and the at least one end effector.

In particular, the method for calibrating a tendon-driven wearable orthosis may comprise any combination of the features described herein for a tendon-driven wearable orthosis.

A further aspect of the disclosure relates to a method for assisting a movement of at least one joint of a user. The method comprises calibrating a tendon-driven wearable orthosis configured to be worn by the user. In particular, the step of calibrating the tendon-driven wearable orthosis may comprise performing the method for calibrating a tendon-driven wearable orthosis described herein. The method for assisting a movement of at least one joint of a user further comprises releasably connecting the adapter unit to a motor unit configured to operate the adapter unit to exert a pulling force on the at least one tendon to assist the movement of the at least one joint of the user and operating the adapter unit using the motor unit.

The step of releasably connecting the adapter unit may further comprise releasably connecting the adapter unit to the motor unit while the at least one tensioning tool is connected to the adapter unit, and disconnecting the at least one tensioning tool from the adapter unit. The at least one tensioning tool may, for example, be at least one first tensioning tool, at least one second tensioning tool, and/or at least one third tensioning tool, as described herein.

In particular, the method for assisting a movement of at least one joint of a user may comprise any combination of the features described herein for a tendon-driven wearable orthosis.

The invention will now be further discussed and explained by reference to exemplary embodiments illustrated in the appended Figures. It is to be understood that the invention is not limited to said exemplary embodiments, but instead may comprise any combination of features described herein and/or shown in the appended Figures.

The Figures show:
- **Figure 1:**: a top view of an exemplary adapter top plate of an exemplary adapter unit;
- **Figure 2:**: a bottom view of an exemplary adapter top plate of an exemplary adapter unit;
- **Figure 3**:: a top view of an exemplary adapter bottom plate of an exemplary adapter unit;
- **Figures 4A to 4B:**: a perspective view of an exemplary first spool from a front side and a back side, respectively;
- **Figures 5A to 5B:**: a perspective view of an exemplary first tensioning tool from a front side and a back side, respectively;
- **Figures 6A to 6B:**: an exploded, perspective view of an exemplary adapter unit from a front and back side, respectively;
- **Figures 7A to 7D:**: an exemplary adapter unit in combination with an exemplary motor unit in different configurations while adjustably controlling the length of the at least one tendon;
- **Figure 8:**: an abstract, schematic overview of a tendon-driven wearable orthosis;
- **Figures 9A to 9B:**: two perspective views of an exemplary third spool of an exemplary multi-spool unit;
- **Figures 10 to 11:**: two perspective views, respectively, of an exemplary multi-spool unit;
- **Figures 12 to 15:**: perspective views of the multi-spool unit in different configurations;
- **Figure 16:**: a perspective, exploded view of a further exemplary adapter unit with a further multi-spool unit;
- **Figure 17:**: a perspective, partially exploded view of a multi-spool unit and adapter unit;
- **Figure 18:**: a perspective view of an exemplary adapter unit;
- **Figures 19A to 19B:**: a perspective top view and bottom view of an adapter unit, respectively;
- **Figure 20:**: a schematic side-view of a further exemplary adapter unit and exemplary motor unit;
- **Figure 21:**: a schematic top view of an exemplary adapter unit;
- **Figure 22:**: a flow diagram of an exemplary method for calibrating a tendon-driven wearable orthosis configured to be worn by a user; and
- **Figure 23:**: a flow diagram of an exemplary method for assisting a movement of at least one joint of a user.

It is noted that in several of the following Figures, the at least one tendon and/or the at least one end effector have been omitted from illustration. This is not to be understood as restricting the scope of the disclosure: Instead, omission of the at least one tendon and/or the at least one end effector has been undertaken solely for illustrative purposes.

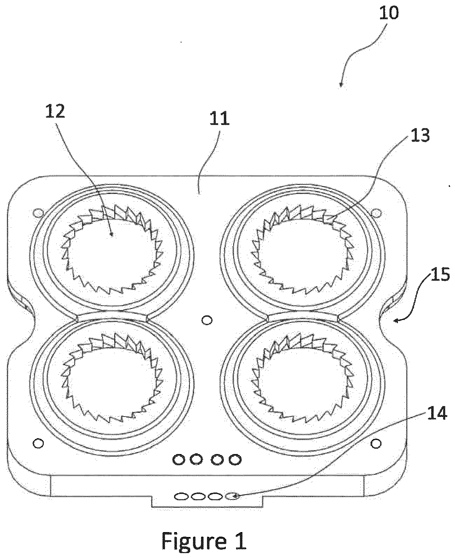

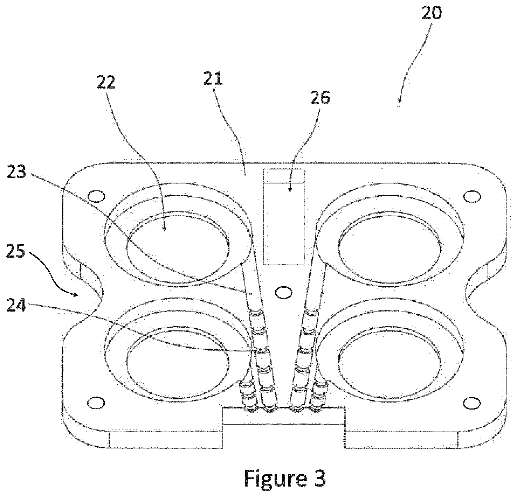

**Figure 1** shows a top view of an exemplary adapter top plate **10** of an exemplary adapter unit **1.** In particular the adapter unit **1** may comprise the adapter top plate **10** and an adapter bottom plate **20** (shown in, e.g., Figure 3), wherein the adapter top plate **10** and the adapter bottom plate **20** may be fixed to one another to form a housing of the adapter unit **1.**

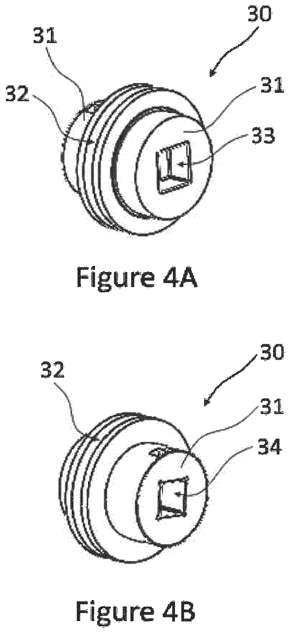

The adapter top plate **10** may be substantially planar and comprise a top surface **11** and a plurality of top spool bores **12.** In the present exemplary adapter top plate **10** only four top spool bores **12** are shown. It is to be understood, however, that more or less top spool bores **12** may be provided. Each of the plurality of top spool bores **12** may be configured to receive at least part of a correspondingly shaped first spool **30** (shown in, e.g., Figure 4A). Each of the plurality of top spool bores **12** may be configured to substantially extend through the adapter top plate **10** in a direction substantially orthogonal to the top surface **11.** Each top spool bore **12** may further comprise a plurality of second ratcheting teeth **13** arranged along an edge of the respective top spool bore **12** at the top surface **11.** Each of the plurality of second ratcheting teeth **13** may extend at least partially along a radial direction of the respective top spool bore **12** towards the centre of said top spool bore **12.** Each second ratcheting teeth **13** may further comprise a blocking contact surface substantially parallel to the radial direction and an angled contact surface substantially at a non-zero angle to said radial direction. While an exemplary shape of each of the plurality of second ratcheting teeth has been described above, it is noted that each of the plurality of first, third, and fourth ratcheting teeth may be similarly and/or correspondingly engageably shaped. However, such an exemplary shape is not to be understood as limiting. Instead, different exemplary shapes may be implemented, and may be dependent on the first, second, third, and/or fourth rotation directions.

The adapter top plate **10** may further comprise a plurality of tendon bores **14** configured to each guide at least one tendon from one or more top spool bores **12** at least partially towards at least one end effector. The tendon bores **14** may extend substantially parallel to one another and/or substantially orthogonally to the plurality of top spool bores **12.** However, the disclosure is not limited to such a configuration, and different arrangements of the tendon bores **14** may be provided, such as according to individual requirements. Furthermore, the tendon bores **14** may be provided in a part of the adapter top plate **10** having a thickness larger than an average thickness of the adapter top plate **10.**

Furthermore, the adapter top plate **10** may further comprise at least one top handling recess **15.** The at least one top handling recess **15** may extend substantially orthogonally from a side surface of the adapter top plate **10.** The at least one top handling recess **15** may be configured to facilitate handling and/or improve usability of the adapter top plate **10** and/or the adapter unit **1** by a user.

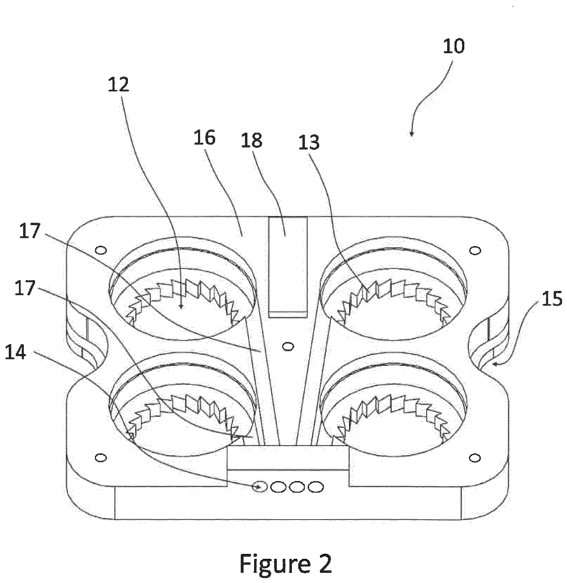

**Figure 2** shows a bottom view of an exemplary adapter top plate **10** of an exemplary adapter unit **1.**

As shown in Figure 2, the plurality of top spool bores **12** may extend through the adapter top plate from the top surface **11** to a bottom surface **16** of the adapter top plate **10.** Top surface **11** and bottom surface **16** may be substantially parallel to one another. Furthermore, as illustrated in Figure 2, the plurality of second ratcheting teeth **13** may be provided in each top spool bore **12** only in a section substantially adjacent to the top surface **11.**

Furthermore, a plurality of top tendon grooves **17** may be provided in the bottom surface **16.** Each top tendon groove **17** may be configured to extend from one of the top spool bores **12** to one of the tendon bores **14,** and may be configured to guide a respective tendon from a top spool bore **12** to said one of the tendon bores **14.**

In addition, the adapter top plate **10** may further comprise an alignment protrusion **18** extending from the bottom surface **16.** While the alignment protrusion **18** shown in Figure 2 is substantially rectangular, differently shaped alignment protrusions **18** may be provided. The alignment protrusion **18** may in particular be configured to enter into engagement with a corresponding alignment recess **26** (see, e.g., Figure 3) of the adapter bottom plate **20** in order to align adapter top plate **10** and adapter bottom plate **20** with respect to one another.

**Figure 3** shows a top view of an exemplary adapter bottom plate **20** of an exemplary adapter unit **1.**

The adapter bottom plate **20** comprises a top surface **21** and a plurality of bottom spool bores **22.** In the present exemplary adapter bottom plate **20** only four bottom spool bores **22** are shown. It is to be understood, however, that more or less bottom spool bores **22** may be provided. Each of the plurality of bottom spool bores **22** may be configured to receive at least part of a correspondingly shaped first spool **30** (shown in, e.g., Figure 4A). Each of the plurality of bottom spool bores **22** may be configured to substantially extend through the adapter bottom plate **20** in a direction substantially orthogonal to the top surface **21.**

Furthermore, the plurality of top spool bores **12** and the plurality of bottom spool bores **22** may be provided such that each top spool bore **12** aligns with one bottom spool bore **22** to form a receptacle space, in which a respective first spool **30** may be accommodated and/or inserted.

A plurality of bottom tendon grooves **23** may be provided in the top surface **21.** Each bottom tendon groove **23** may be configured to extend from one of the bottom spool bores **22** towards one of the tendon bores **14,** and may be configured to guide a respective tendon from a bottom spool bore **22** to said one of the tendon bores **14.** In particular, the plurality of top tendon grooves **17** and the plurality of bottom tendon grooves **23** may be provided such that each top tendon groove **17** aligns with a bottom tendon groove **23** to define a guide passage for a respective tendon, wherein each guide passage extends from one of the receptacle spaces to one of the tendon bores **14.**

Each bottom tendon groove **23** may further comprise one or more groove protrusions **24,** preferentially one or more semi-circular groove protrusions **24,** arranged at one or more locations along the respective bottom tendon groove **23.** By providing such groove protrusions **24,** a contact area between a tendon and the respective bottom tendon groove **23** may be reduced, thereby facilitating movement of said tendon in said bottom tendon groove **23** and further reducing wear-and-tear thereon. Furthermore, it is noted that any number of groove protrusions **24** may be provided. In addition, the plurality of groove protrusions **24** may be provided in one or more of the plurality of bottom tendon grooves **23** and/or in one or more of the top tendon grooves **17.**

The adapter bottom plate **20** may further comprise at least one bottom handling recess **15.** The at least one bottom handling recess **25** may extend substantially orthogonally from a side surface of the adapter bottom plate **20.** The at least one bottom handling recess **25** may be configured to facilitate handling and/or improve usability of the adapter bottom plate **20** and/or the adapter unit 1 by a user. Furthermore, at least one top handling recess **15** may be provided to be substantially aligned with at least one bottom handling recess **25** to form a common handling recess, when the adapter top plate **10** and the adapter bottom plate **20** are connected to one another.

The adapter bottom plate **20** may further comprise an alignment recess **26** extending from the top surface **21.** The alignment recess **26** may in particular be configured to enter into engagement with a corresponding alignment protrusion **18** (see, e.g., Figure 2) of the adapter top plate **10** in order to align adapter top plate **10** and adapter bottom plate **20** with respect to one another. The invention, however, is not limited to the exemplary alignment recess **26** and alignment protrusion **18.** Instead, each of the adapter top plate **10** and the adapter bottom plate **20** may comprise one or more alignment recesses **26** and/or one or more alignment protrusions **18** in order to align the adapter top plate **10** and the adapter bottom plate **20.** Furthermore, a shape of each of the alignment recesses **26** and/or of each of the alignment protrusions **18** may be freely chosen, wherein the at least one alignment recess **26** is configured to enter into engagement with at least one alignment protrusion **18** to align adapter top plate **10** and adapter bottom plate **20** with respect to one another.

**Figures 4A** and **4B** show a perspective view of an exemplary first spool **30** from a front side and a back side, respectively.

The first spool **30** comprises a substantially cylindrical central rotation shaft **31** and a tendon winding section **32.** The tendon winding section **32** may be arranged substantially in the middle of the central rotation shaft **31,** wherein at least one tendon is connectable to the first spool **30** in the tendon winding section **32.** In particular, the tendon winding section **32** may be configured such that a tendon connected thereto may be wound around at least the central rotation shaft **31** (or unwound therefrom, respectively) by rotation of the first spool **30** around the central rotation shaft **31** and/or a central rotation axis. The central rotation axis may correspond to a central axis of the central rotation shaft **31.** The tendon winding section **32** may furthermore comprise a first circumferential bordering wall and a second circumferential bordering wall, wherein a tendon is connectable to the first spool **30** between the first circumferential bordering wall and the second circumferential bordering wall.

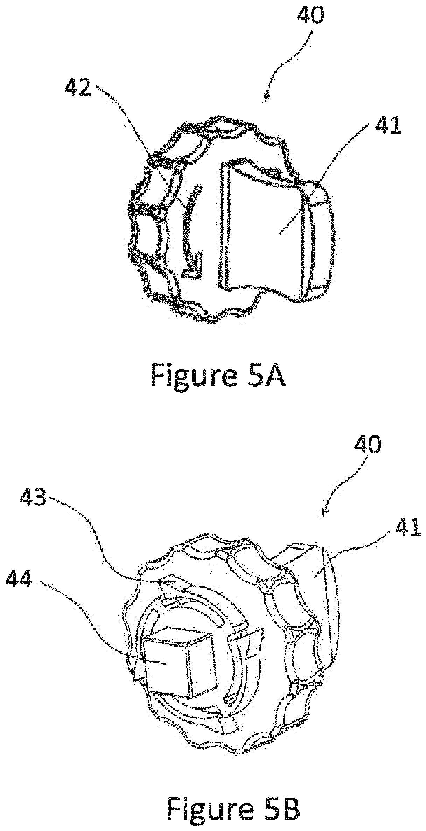

The first spool **30** may further comprise a first tensioning tool engagement section **33.** The first tensioning tool engagement section **33** may be configured to be engageable with a first tensioning tool, such as a ratcheting key **40** (as shown, e.g., in Figures 5A and 5B). The first tensioning tool engagement section **33** may be arranged at a first end of the central rotation shaft **31,** and may further be configured to be engageable with the first tensioning tool such that rotation of the first tensioning tool in engagement with the first tensioning tool engagement section **33** causes a rotation of the first spool **30** around the central rotation shaft **31** and/or the central rotation axis.

In the illustrated exemplary embodiment of the first spool **30,** the first tensioning tool engagement section **33** is shown as a substantially cubic recess. However, the first tensioning tool engagement section **33** is not restricted to such an implementation. In particular, the first tensioning tool engagement section **33** may comprise one or more engagement recesses and/or one or more engagement protrusions. Furthermore, a shape of each of the one or more engagement recesses and/or one or more engagement protrusions maybe freely chosen, for example according to the specific requirements of the user.

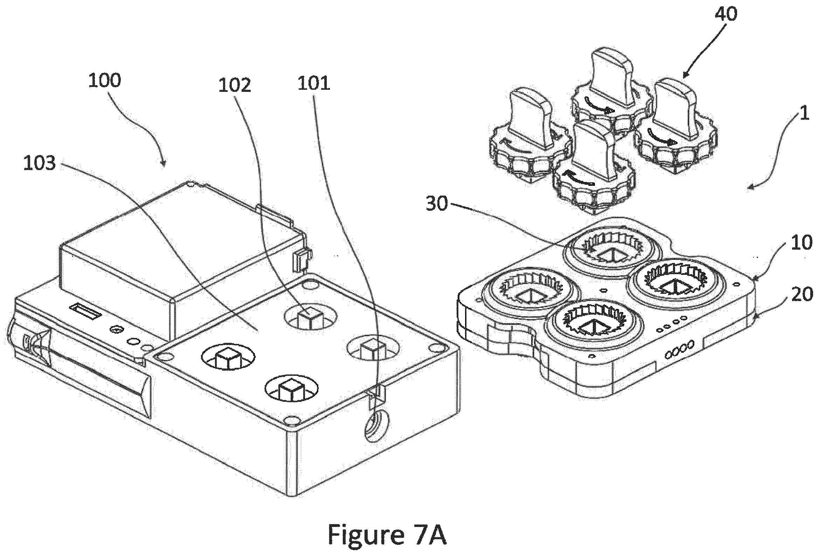

The first spool **30** may further comprise a first motor unit engagement section **34.** The first motor unit engagement section **34** may be configured to be engageable with a motor unit **100** and/or a motor unit spool engagement section **102,** as for example shown in Figure 7A. The first motor unit engagement section **34** may be arranged at a second end of the central rotation shaft **31,** and may further be configured to be engageable with the motor unit **100** and/or the motor unit spool engagement section **102** such that rotation of the motor unit **100** and/or the motor unit spool engagement section **102** in engagement with the first motor unit engagement section **34** causes a rotation of the first spool **30** around the central rotation shaft **31** and/or the central rotation axis.

In the illustrated exemplary embodiment of the first spool **30,** the first motor unit engagement section **34** is shown as a substantially cubic recess. However, the first motor unit engagement section **34** is not restricted to such an implementation. In particular, the first motor unit engagement section **34** may comprise one or more engagement recesses and/or one or more engagement protrusions. Furthermore, a shape of each of the one or more engagement recesses and/or one or more engagement protrusions maybe freely chosen, for example according to the specific requirements of the user.

**Figures 5A** and **5B** show a perspective view of an exemplary first tensioning tool configured as an exemplary ratcheting key **40** from a front side and a back side, respectively.

The ratcheting key **40** may comprise a handle **41** provided at a first end of the ratcheting key **40,** wherein the handle **41** is configured to facilitate rotation of the ratcheting key **40** round a rotation axis thereof. Please note that the handle **41** is not restricted to the arrangement and shape shown in Figures 5A and 5B, but may be freely chosen, such as according to specific requirements of the user.

The ratcheting key **40** may further comprise one or more rotation direction indicators **42,** wherein the one or more rotation direction indicators **42** may indicate an allowed rotation direction of the ratcheting key **40** when the ratcheting key **40** is engaged with at least the adapter unit **1.**

The ratcheting key **40** may further comprise a plurality of first ratcheting teeth **43.** The first ratcheting teeth **43** may be configured as a series of first ratcheting teeth **43** arranged circumferentially around a rotation axis of the ratcheting key **40,** and may further each comprise at least one protrusion extending radially outward with respect to the rotation axis. Furthermore, each of the ratcheting teeth **43** may be configured to be elastically deformable, wherein each of the ratcheting teeth **43** may be configured to be elastically displaceable along a radial direction, such as elastically displaceable inward along the radial direction, with respect to the rotation axis.

The ratcheting key **40** may further comprise a first spool engagement section **44.** The first spool engagement section **44** may be configured to be engageable with a first spool **30** and/or a first tensioning tool engagement section **33** of the first spool **30,** as for example shown in Figure 4A. The first spool engagement section **44** may be arranged at a second end of the ratcheting key **40,** and may further be configured to be engageable with the first spool **30** and/or the first tensioning tool engagement section **33** of the first spool **30** such that rotation of the ratcheting key **40** in engagement with the first spool **30** and/or the first tensioning tool engagement section **33** causes a rotation of the first spool **30** around the central rotation shaft **31** and/or the central rotation axis thereof.

In the illustrated exemplary embodiment of the ratcheting key **40,** the first spool engagement section **44** is shown as a substantially cubic protrusion. However, the first spool engagement section **44** is not restricted to such an implementation. In particular, the first spool engagement section **44** may comprise one or more engagement recesses and/or one or more engagement protrusions. Furthermore, a shape of each of the one or more engagement recesses and/or one or more engagement protrusions maybe freely chosen, for example according to the specific requirements of the user.

The exemplary ratcheting key **40** may be configured to be releasably engageable with the adapter unit **1** and at least one first spool **30** to bring the plurality of first ratcheting teeth **43** into engagement with at least a subset of the plurality of second ratcheting teeth **13.** The plurality of first ratcheting teeth **43** and at least the subset of plurality of second ratcheting teeth **13** may be configured to prevent rotation of the at least one first spool **30** and the ratcheting key **40** in a first rotation direction and allow rotation of the at least one first spool **30** and the ratcheting key **40** in a second rotation direction opposite to the first rotation direction, while the plurality of first ratcheting teeth **43** is in engagement with at least the subset of plurality of second ratcheting teeth **13.**

It is noted, however, that the first ratcheting teeth **43** according to the disclosure are not to be understood as restricted to, for example, the shown number, shape, and configuration of the ratcheting teeth **43** in Figures 5A and 5B.

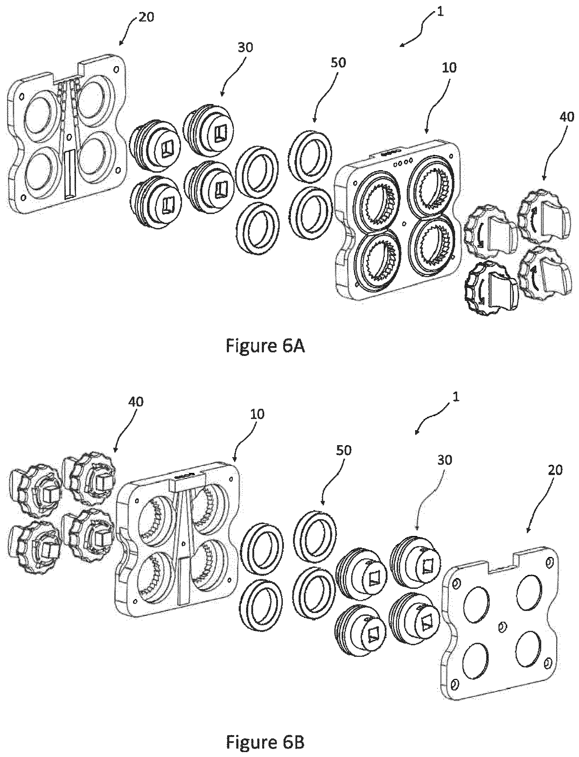

**Figures 6A** and **6B** show an exploded, perspective view of an exemplary adapter unit **1** from a front and back side, respectively.

An adapter bottom plate **20** may be provided, as for example shown in Figure 3. The adapter bottom plate **20** may comprise at least one bottom spool bore **22.**

At least one first spool **30** may be provided, as for example shown in Figures 4A and 4B, wherein a first spool **30** may be at least partially received in each bottom spool bore **22** of the adapter bottom plate **20.**

Each first spool **30** may further comprise a spool bearing **50,** wherein the spool bearing **50** may be implemented, for example, as a spool bearing washer. The spool bearing **50** may be releasably mountable on the first spool **30,** such as on a central rotation shaft **31** of the first spool **30,** wherein the spool bearing **50** may be provided substantially between the first spool **30** and the adapter bottom plate **20** and/or the adapter top plate **10.** Although not shown in Figures 6A and 6B, more than one spool bearing **50** may be provided for each first spool **30.**

An adapter top plate **10** may be provided, as for example shown in Figures 1 and 2. The adapter top plate **10** may comprise at least one top spool bore **12.** Each first spool **30** and spool bearing **50** may be at least partially received in a corresponding top spool bore **12.**

The adapter top plate **10** and the adapter bottom plate **20** may be fixed to one another to form a housing of the adapter unit **1.** Furthermore, ratcheting keys **40** may be provided, as for example shown in Figures 5A and 5B. In particular, a ratcheting key **40** may be provided for each first spool **30.**

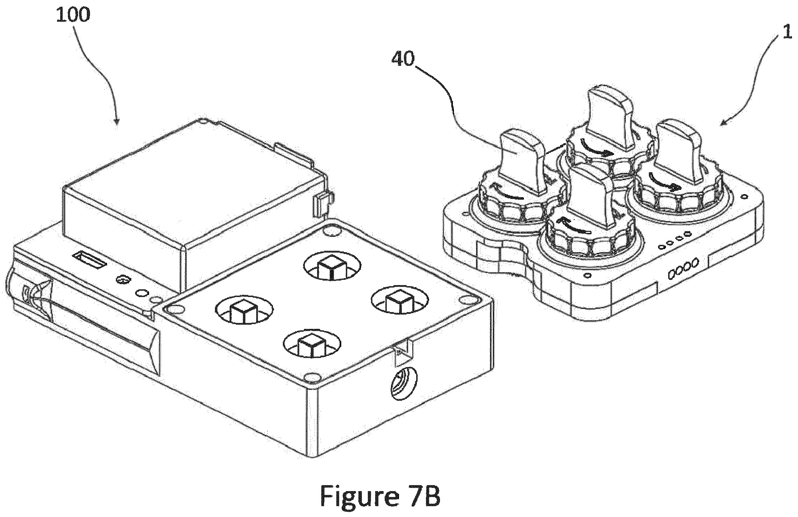

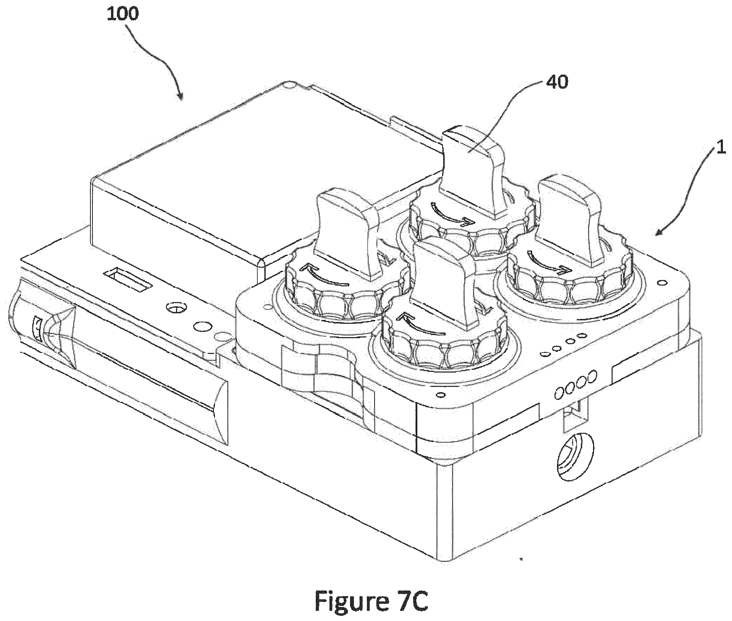

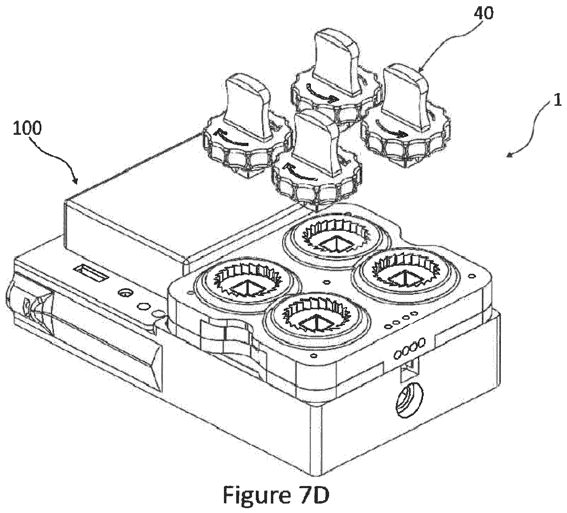

**Figures 7A****,** **7B****,** **7C****,** and **7D** show an exemplary adapter unit **1** in combination with an exemplary motor unit **100** in different configurations while adjustably controlling the length of the at least one tendon.

The motor unit **100** may comprise a housing configured to house an actuator, such as a rotary actuator, and a drive shaft **101.** The drive shaft **101** may be configured to be operable, such as rotatable, by the actuator. The drive shaft **101** may further be configured to operate and/or rotate one or more motor unit spool engagement sections **102.** Each motor unit spool engagement section **102** may be configured to be engageable with a first motor unit engagement section **34** of a corresponding first spool **30.**

The motor unit **100** may further comprise an adapter mounting surface **103,** wherein the adapter unit **1** and/or the bottom adapter plate **20** may be mountable on the adapter mounting surface **103** such that each motor unit spool engagement section **102** enters into engagement with a corresponding first motor unit engagement section **34.**

As shown in Figure 7A, in an initial state, the adapter unit **1** is provided separate from the motor unit **100,** wherein the ratcheting keys **40** are provided not in engagement with the adapter unit **1.** It is noted that the tendon-driven wearable orthosis may be mounted, in a first step, on the user. For illustrative purposes, such a step is not shown in Figure 7A.

In a second step, shown in Figure 7B, the ratcheting keys **40** may be mounted on the adapter unit **1** to enter into engagement with the adapter unit **1** and/or the at least one first spool **30.** Specifically, the ratcheting keys **40** may be mounted on the top surface **11** of the top adapter plate **10.** By turning the ratcheting keys **40,** the length of each tendon connected to the first spools **30** may be individually controlled and/or adjusted. For example, in the second step, the ratcheting keys **40** may be turned to adjust, preferably to reduce, the length of each tendon connected to the first spools **30** to a respective desired length.

In a third step, shown in Figure 7C, the adapter unit **1** may be mounted on the motor unit **100,** while the ratcheting keys **40** remain mounted on the adapter unit **1.** In particular, it therefore is possible to maintain the adjusted and desired lengths of the at least one tendon until at least the adapter unit **1** is mounted on the motor unit **100.** For example, during the third step, the ratcheting keys **40** may be further turned to adjust, preferably to further reduce, the length of each tendon connected to the first spools **30** to allow each motor unit spool engagement section **102** to enter into engagement with a corresponding first motor unit engagement section **34.**

In a fourth step, shown in Figure 7D, the ratcheting keys **40** may be removed and/or unmounted form the adapter unit **1.** By removing the ratcheting keys **40,** the motor unit **100** may freely operate the adapter unit **1** to exert a pulling force on the at least one tendon to assist the movement of the user.

Figure 8 shows an abstract, schematic overview of a tendon-driven wearable orthosis. The tendon-driven wearable orthosis may comprise an adapter unit **1** and a motor unit **100,** as described herein, wherein the adapter unit **1** is releasably connectable to the motor unit **100.** Furthermore, the adapter unit **1** may be connectable, via at least one tendon **60,** to at least one end effector **70.** The adapter unit **1** may comprise the at least one tendon **60** and/or the at least one end effector **70.**

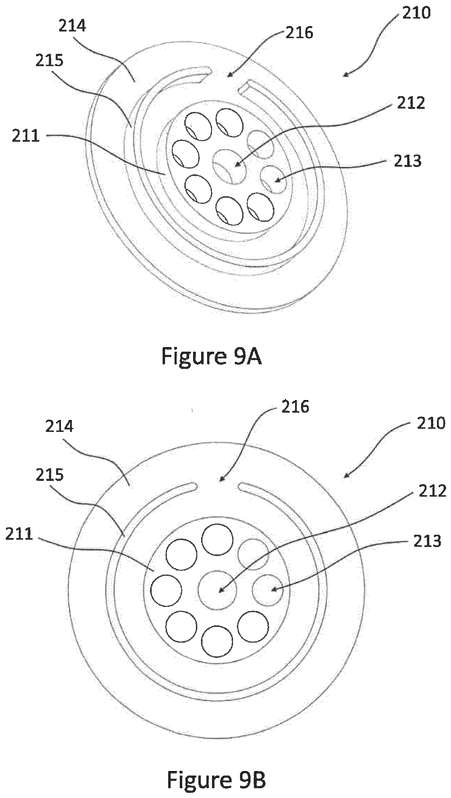

**Figures 9A** and **9B** show two perspective views of an exemplary third spool **210** of an exemplary multi-spool unit **200.**

The third spool **210** may comprise a cylindrical central section **211,** wherein the cylindrical central section **211** may comprise a central mounting bore **212.** The cylindrical central section **211** may further comprise one or more locking bores **213,** wherein the one or more locking bores **213** may be arranged adjacent to the central mounting bore **212.**

The one or more locking bores **213** may be configured as a single locking bore **213** (not shown in Figures 9A and 9B) or as a plurality of locking bores **213,** wherein the plurality of locking bores **213** may be arranged, and preferentially equally spaced, to circularly surround the central mounting bore **212.** The central mounting bore **212** and the one or more locking bores **213** may further extend substantially parallel to one another through the cylindrical central section **211.**

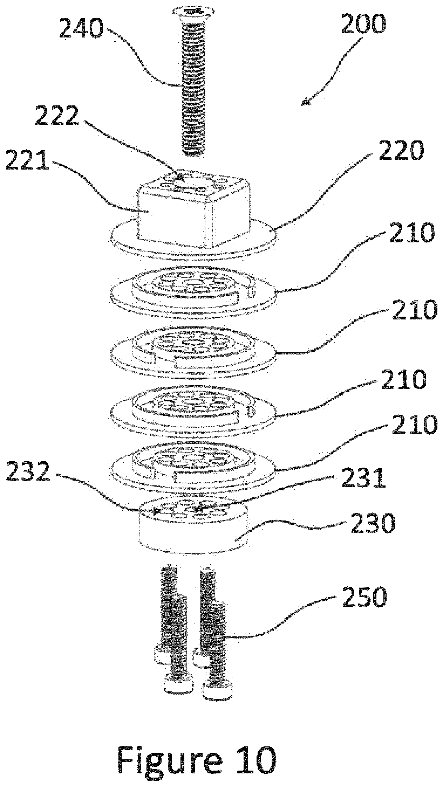

The third spool **210** may further each comprise a, preferably circular, mounting plate **214** extending from the cylindrical central section **211** outwards. The mounting plate **214** may be substantially planar and orthogonal to a central axis of the central mounting bore **212.** The mounting plate **214** may further comprise a circumferential wall **215,** wherein the circumferential wall **215** is configured to extend orthogonally from the mounting plate **214** and at least partially surround the cylindrical central section **211.** The circumferential wall **215** may comprise at least one opening **216,** wherein the opening **216** is configured to allow at least one tendon, fixed to the cylindrical central section **211,** to pass through the circumferential wall **215.** Each third spool **210** may be configured such that, during rotation of the third spool **210** around a central rotation shaft **240** (see, e.g., Figure 10), a tendon fixed to the respective cylindrical central section **211** may be wound around the circumferential wall **215.**

The mounting plate **214,** the circumferential wall **215,** and at least one of: an adjacent mounting plate **214** of a further third spool **210** mounted adjacent to the exemplary third spool **210,** the top plate **220,** and the bottom plate **230,** may be configured to form a tendon channel for at least one tendon fixed to the cylindrical central section **211.** Specifically, the tendon channel may be configured such that, during rotation of the exemplary third spool **210,** a tendon fixed to the respective cylindrical central section **211** may be wound around the circumferential wall **215** within the tendon channel.

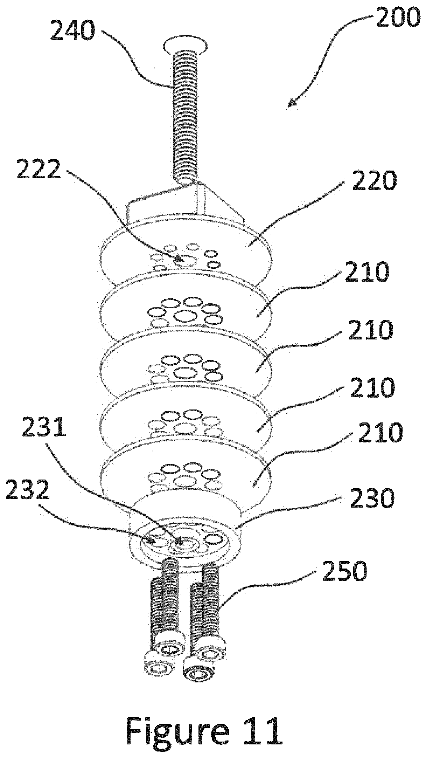

**Figures 10** and **11** show two perspective views, respectively, of an exemplary multi-spool unit **200.**

The multi-spool unit **200** may comprise at least a central rotation shaft **240** and a plurality of third spools **210** mountable on the central rotation shaft **240.** Each third spool **210** may be mountable on the central rotation shaft **240** substantially adjacent to at least one other third spool **210.** The central rotation shaft **240** may be implemented as a central shaft screw. Each third spool **210** may comprise a central mounting bore **212** extending through the respective third spool **210,** wherein the central rotation shaft **240** is insertable into the central mounting bore **212.** Each third spool **210** may be configured as a third spool **210** shown in Figures 9A and 9B. Furthermore, the plurality of third spools **210** may be substantially identical to one another, or the plurality of third spools **210** may comprise two or more third spools **210** different to one another.

The multi-spool unit **200** may further comprise a top plate **220** mountable on the central rotation shaft **240.** The top plate **220** may comprise a central top plate mounting bore **222** extending through the top plate **220,** wherein the central rotation shaft **240** is insertable into the central top plate mounting bore **222.** The top plate **220** may further comprise one or more top plate locking bores configured to be releasably engageable with a third tensioning tool **250.** Furthermore, the top plate **220** may in addition comprise a top plate motor engagement section **221,** wherein the top plate motor engagement section **221** may be configured to be engageable with a motor unit to allow the motor unit to operate, preferentially to rotate, the multi-spool unit **200.** A shape of the top plate motor engagement section **221** may be chosen according to, for example, the specific requirements of the user and/or the motor unit.

The multi-spool unit **200** may further comprise a bottom plate **230** mountable on the central rotation shaft **240.** The bottom plate **230** may comprise a central bottom plate mounting bore **231** extending through the bottom plate **230,** wherein the central rotation shaft **240** is insertable into the central bottom plate mounting bore **231.** The central rotation shaft **240** may be engageable with the central bottom plate mounting bore **231,** such as by screwing the central rotation shaft **240** into the central bottom plate mounting bore **231.** Furthermore, the plurality of third spools **210** may be mountable on the central rotation shaft **240** between the top plate **220** and the bottom plate **230.** Specifically, the central rotation shaft **240** may be engageable with the central bottom plate mounting bore **231,** to press the top plate **220** and the bottom plate **230** together, thereby generating a compression force and/or squeezing force on the plurality of third spools **210.**

In particular, the multi-spool unit **200** may be configured to be switchable between a first state and a second state. In the first state, each of the plurality of third spools **210** may be configured to be rotatable, preferentially freely rotatable, around the central rotation shaft **240.** In the second state, the plurality of third spools **210** may be configured to be fixed relative to the central rotation shaft **240** by compressing the plurality of third spools **210** between the top plate **220** and the bottom plate **230.** In the second state, the multi-spool unit **200** may be configured to be rotatable by the motor unit to exert the pulling force on the at least one tendon.

The bottom plate **230** may further comprise one or more bottom plate locking bores **232** configured to be releasably engageable with at least one third tensioning tool **250.** The at least one third tensioning tool **250** may be implemented as at least one locking screw, wherein each locking screw may be engageable with at least one bottom plate locking bore **232.** In addition, the bottom plate **230** may further comprise a recess configured to at least partially receive the at least one third tensioning tool **250,** such as for example a screw head of the at least one locking screw.

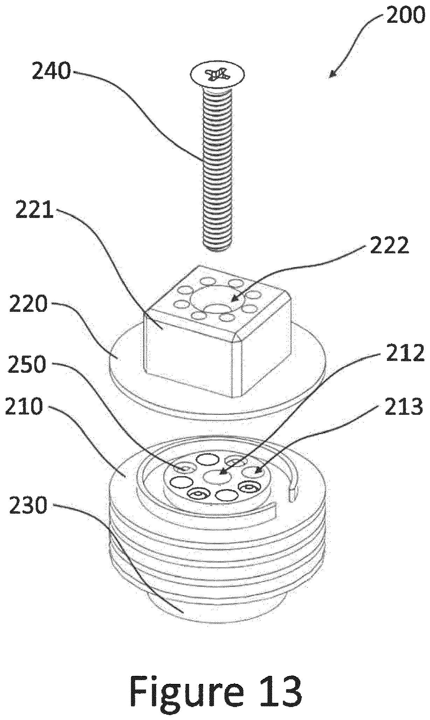

The at least one third tensioning tool **250** may be configured to be releasably engageable with the plurality of third spools **210** in at least the first state to fix an orientation of the plurality of third spools **210** relative to one another. A third tensioning tool **250** may for example be configured to be inserted into a bottom plate locking bore **232** of the bottom plate **230** and a locking bore **213** of each of the plurality of third spools **210** to fix an orientation of the plurality of third spools **210** relative to one another. Such a configuration has been illustrated, for example, in **Figure 13****,** wherein the top plate **220** and the central rotation shaft **240** have been illustratively displaced to enable a clearer view.

The at least one third tensioning tool **250** may be removable from the multi-spool unit **200** in the second state, wherein a compressive force generated by pressing the top plate **220** and bottom plate **230** together, as described above, may be sufficient to prevent a rotation of the third spools **210** relative to one another in the second state. Alternatively, the at least one third tensioning tool **250** may be maintained in the multi-spool unit **200** in the second state to ensure no relative rotation between the plurality of third spools **210.**

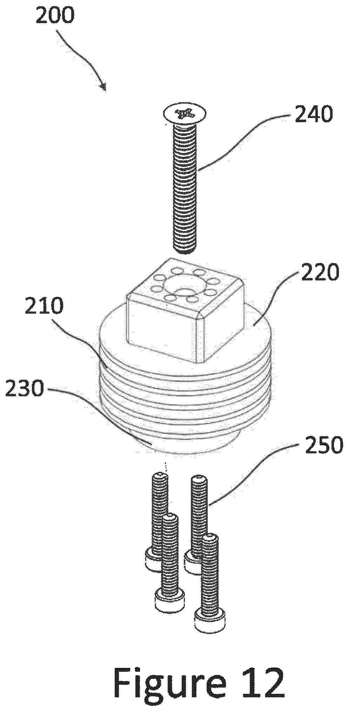

**Figure 12** shows a partially assembled view of the multi-spool unit **200.** In the shown view, the top plate **220,** the plurality of third spools **210,** and the bottom plate **230** are arranged adjacent to one another in a stack configuration, while the central rotation shaft **240** and the at least one third tensioning tool **250** are illustrated separately form the stack configuration.

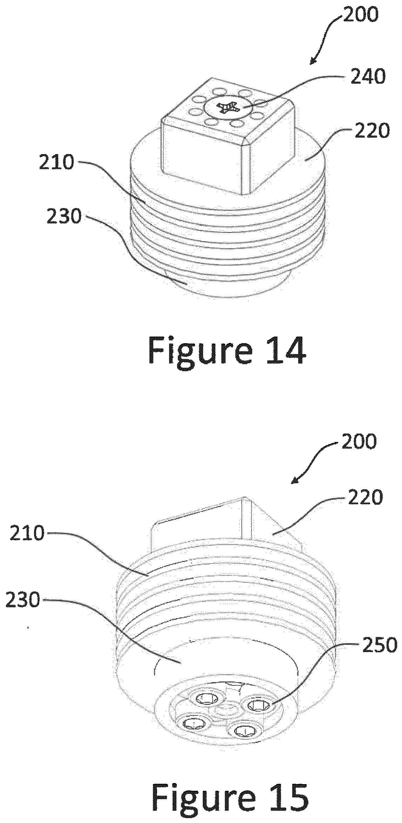

**Figures 14** and **15** show two perspective views of a multi-spool unit **200** according to one of Figures 10 to 13 in a fully assembled configuration.

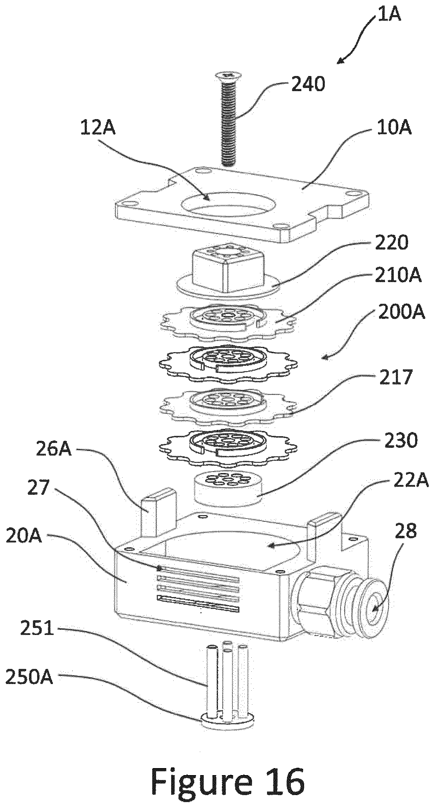

**Figure 16** shows a perspective, exploded view of a further exemplary adapter unit **1A** with a further multi-spool unit **200A.**

The adapter unit **1A** may comprise a top plate **10A,** wherein the top plate **10A** may be substantially planar. A multi-spool unit recess **12A** may be provided in the top plate **10A** and configured to at least partially receive and/or accommodate the multi-spool unit **200A.**

The adapter unit **1A** may further comprise a bottom plate **20A.** The bottom plate **20A** may comprise a multi-spool accommodation space **22A** configured to at least partially accommodate the multi-spool unit **200A.** The multi-spool unit recess **12A** and the multi-spool accommodation space **22A** may be configured to form a substantially closed accommodation space, in which the multi-spool unit **200A** may be accommodated. Furthermore, the top plate **10A** and the bottom plate **20A** may be configured to form a housing for the multi-spool unit **200A.** The bottom plate **20A** may further comprise one or more alignment posts **26A** configured to at least partially engage the top plate **10A** to align the top plate **10A** with respect to the bottom plate **20A.**

In addition, the bottom plate **20A** may further comprise a tendon outlet **28,** wherein the tendon outlet **28** is configured to guide one or more tendons connected to the multi-spool unit **200A** from within the multi-spool accommodation space **22A** through the bottom plate **20A** towards the at least one end effector. The tendon outlet **28** may be implemented, for example, as one or more outlet bores in the bottom plate **20A.**

The multi-spool unit **200A** may further comprise a central rotation shaft **240,** a top plate **220,** and a bottom plate **230** substantially similar to the central rotation shaft **240,** the top plate **220,** and the bottom plate **230,** respectively, as described for the multi-spool unit **200** above. A duplicate description thereof will therefore be omitted. Similar to the multi-spool unit **200,** the multi-spool unit **200A** may be switchable between the first state and the second state.

The multi-spool unit **200A** may further comprise a plurality of third spools **210A,** wherein the plurality of third spools **210A** may be configured similar to the plurality of third spools **210,** as described for the multi-spool unit **200** above. However, each third spool **210A** may differ from the third spools **210** described above in the provision of additional gripping protrusions **217.** Each third spool **210A** may comprise a plurality of such gripping protrusions **217** arranged around the circumference of the mounting plate **214** and/or the third spool **210A.**

The plurality of gripping protrusions **217** may be configured to facilitate the rotation of each third spool **210A,** such as for example by the user. Furthermore, the bottom plate **20A** may comprise a plurality of access slits **27** through the bottom plate **20A** arranged adjacent the multi-spool accommodation space **22A.** Each access slit **27** may be configured and arranged to at least partially accommodate the plurality of gripping protrusions **217** of a third spool **210A** when the multi-spool unit **200A** is accommodated within the multi-spool accommodation space **22A.** In particular, the plurality of gripping protrusions **217** may therefore at least partially extend from the multi-spool accommodation space **22A** through the access slits **27** when the multi-spool unit **200A** is accommodated within the multi-spool accommodation space **22A.** Thereby, it is easily possible to rotate, for example by hand, each third spool **210A** without having to unmount the top plate **10A** and bottom plate **20A** from one another.

The multi-spool unit **200A** may further differ from the multi-spool unit **200** in the provision of a different third tensioning tool **250A.** The third tensioning tool **250A** may be integrally formed. In particular, the third tensioning tool **250A** may comprise a plurality of locking protrusions **251,** wherein each locking protrusion **251** may be configured to be engageable with a bottom plate locking bore **232** of the bottom plate **230** and a locking bore **213** of each of the plurality of third spools **210A** in order to fix the orientation of the plurality of third spools **210A** with respect to one another.

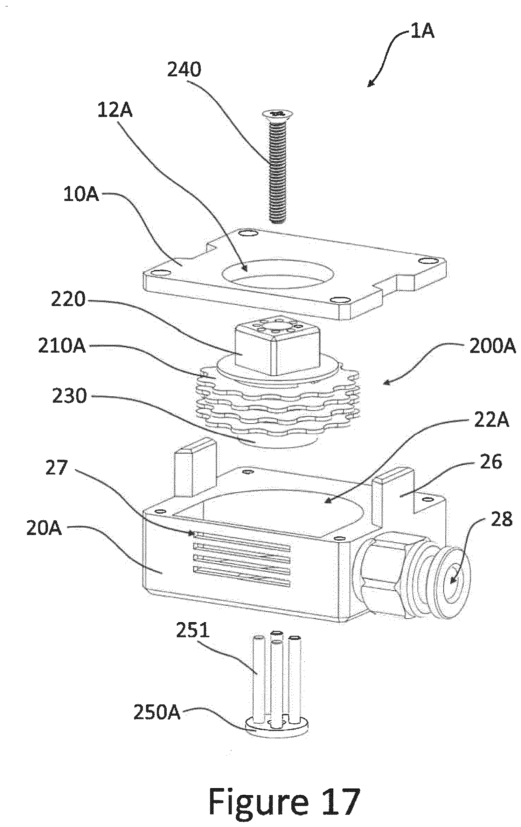

**Figure 17** shows a perspective, partially exploded view of the multi-spool unit **200A** and adapter unit **1A.**

In particular, as shown in Figure 17, the top plate **220,** the plurality of third spools **210A,** and the bottom plate **230** are arranged adjacent to one another in a stack configuration, while the central rotation shaft **240** and the at least one third tensioning tool **250A** are illustrated separately form the stack configuration.

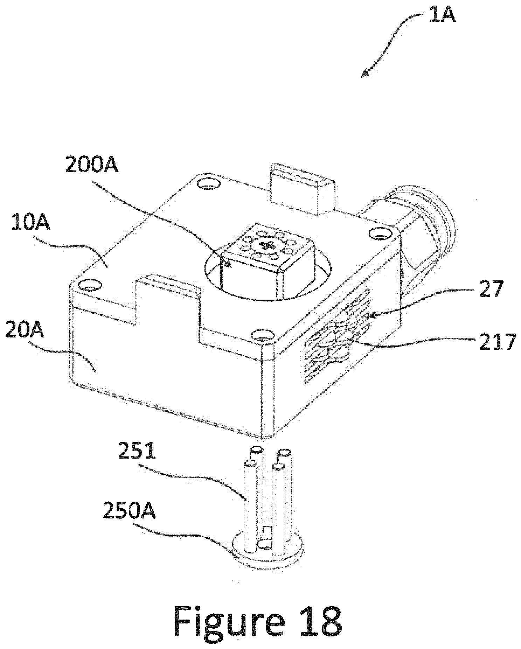

**Figure 18** shows a perspective view of the adapter unit **1A,** wherein the top plate **10A** and the bottom plate **20A** are fixed to one another to accommodate the multi-spool unit **200A.** In the shown adapter unit **1A,** the multi-spool unit **200A** is in the first state and the third tensioning tool **250A** is illustrated separately.

Furthermore, Figure 18 shows a plurality of gripping protrusions **217** of the at least one third spool **210A** extending through the plurality of access slits **27.** In particular, the plurality of gripping protrusions **217** may extend through the plurality of access slits **27** and protrude at least partially from the bottom plate **20A** to be accessible from outside the adapter unit **1A.**

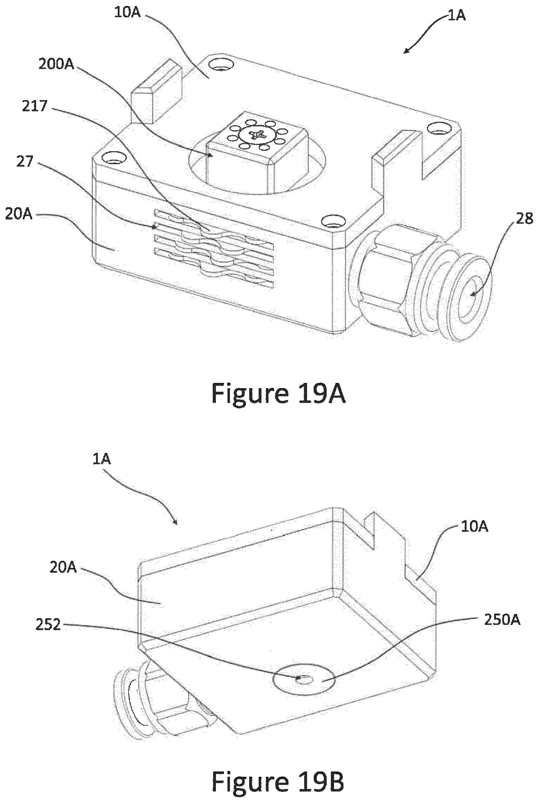

**Figures 19A** and **19B** show a perspective top view and bottom view of the adapter unit **1A,** respectively. In particular, the adapter unit **1A** is shown fully assembled, wherein the third tensioning tool **250A** is brought into engagement with the multi-spool unit **200A** in order to fix the orientation of the plurality of third spools **210A** with respect to one another.

The third tensioning tool **250A** may be configured to be flush with a bottom surface of the bottom plate **20A** when in engagement with the multi-spool unit **200A.** The third tensioning tool **250A** may further comprise an access bore **252.** An access key (not shown) may be provided, wherein the access key may be configured to be engageable with the access bore **252** to remove the third tensioning tool **250A** from the multi-spool unit **200A.** Alternatively, the third tensioning tool **250A** may be provided with a handle (not shown) to allow removal of the third tensioning tool **250A** from the multi-spool unit **200A.**

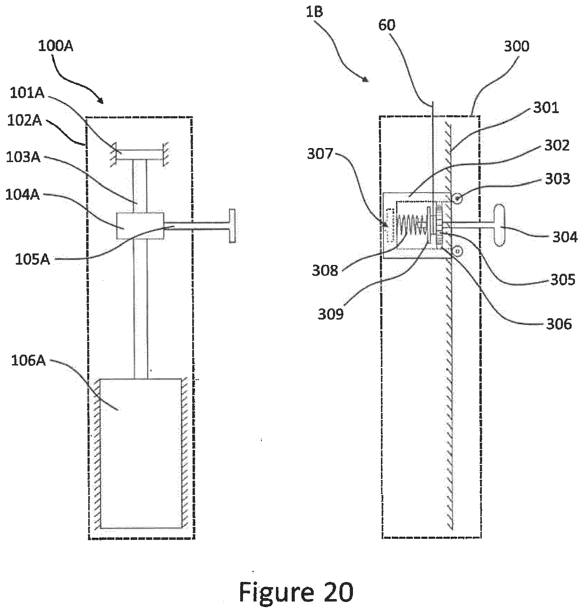

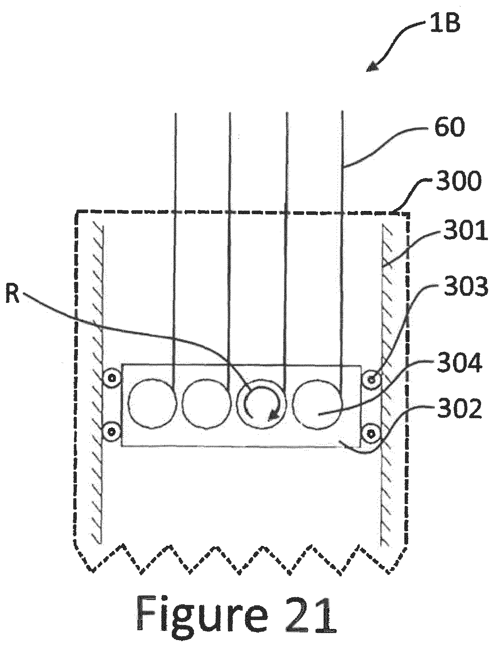

**Figure 20** shows a schematic side-view of a further exemplary adapter unit **1B** and exemplary motor unit **100A,** while **Figure 21** shows a schematic top view of the exemplary adapter unit **1B.**

The adapter unit **1B** and/or the at least one tensioning device may comprise a spool carriage **302** movably mounted on a carriage track **301.** The spool carriage **302** and the carriage track **301** may be provided in a housing **300** of the adapter unit **1B.** The spool carriage **302** may comprise a carriage body and at least one wheel element **303,** wherein the at least one wheel element **303** is fixed to the carriage body and configured to allow the spool carriage **302** to be movable along the carriage track **301.** The carriage track **301** may comprise two guide rails (as seen, for example, in Figure 21), along which the spool carriage **302** may be guided. In the presented exemplary embodiment, the spool carriage **302** may comprise at least two wheel elements **303** for each guide rail.

The at least one tensioning device may further comprise at least one second spool **309** (e.g., four second spools **309,** as shown in Figure 21) mounted on the spool carriage **302,** wherein the at least one tendon **60** is connectable to the at least one second spool **309.** The at least one second spool **309** may be mounted on the spool carriage **302** to be rotatable in a rotation direction **R** (see, e.g., Figure 21) around a central rotation axis of the at least one second spool **309.** One tendon **60** may be at least partially woundable around each second spool **309** by rotation of the respective second spool **309.**

The spool carriage **302** may further comprise a plurality of third ratcheting teeth **306** provided in the spool carriage body of the spool carriage **302.** Each second spool **309** may comprise a plurality of fourth ratcheting teeth **305** releasably engageable with at least a subset of the plurality of third ratcheting teeth **306.** The plurality of fourth ratcheting teeth **305** and at least the subset of plurality of third ratcheting teeth **306** may be configured to prevent rotation of each second spool **309** in a third rotation direction and allow rotation of each second spool **309** in a fourth rotation direction (e.g., rotation direction **R**) opposite to the third rotation direction, while the plurality of fourth ratcheting teeth **305** is in engagement with at least the subset of plurality of third ratcheting teeth **306.**

The at least one tensioning device may further comprise at least one second tensioning tool **304,** wherein each second tensioning tool 304 may be releasably engageable with one second spool **309.** In particular, the at least one second spool **309** may be configured to be rotatable in the rotation direction **R** using the at least one second tensioning tool **304** to control the length of the at least one tendon **60** between the adapter unit **1B** and the at least one end effector.

The at least one second spool **309** may be further configured to be operable using the at least one second tensioning tool **304** to move the plurality of fourth ratcheting teeth **305** out of engagement with at least the subset of the plurality of third ratcheting teeth **306.** In particular, the at least one second spool **309** may comprise a spring element **308.** Specifically, each second spool **309** may thereby be configured as a second spring-loaded spool. The at least one spring element **308** may be fixed at a first spring end to the spool carriage **302** and/or the carriage body, and/or may be fixed at a second spring end to the at least one second spool **309.** A pressing force may be exerted on the second tensioning tool **304** engage with the at least one second spool **309** to cause the at least one spring element **308** to be compressed, thereby moving the plurality of fourth ratcheting teeth **305** out of engagement with at least the subset of the plurality of third ratcheting teeth **306.**

The spool carriage **302** may further be configured to be movable along the carriage track **301** by the motor unit **100A** to exert the pulling force on the at least one tendon **60.**

The motor unit **100A** may comprise a motor element **106A** and a spindle **103A.** The spindle **103A** may be connected, at a first end thereof, to the motor element **106A** and may be connected, at a second end thereof, to a bearing element **101A.** A sled **104A** may be provided on the spindle **103A,** wherein the motor unit **106A** may be configured to operate, e.g., to rotate, the spindle **103A** in order to move the sled **104A** linearly along the spindle **103A.** The motor unit **106A,** the spindle **103A,** the sled **104A,** and the bearing element **101A** may be provided in a motor unit housing **102A.**

The adapter unit **1B** and/or the spool carriage **302** may further comprise a keyed motor engagement section **307,** and the motor unit **100A** and/or the sled **104A** may comprise a keyed connection post **105A.** The keyed connection post **105A** may be configured to be engageable with the keyed motor engagement section **307** when the adapter unit **1B** is connected to the motor unit **100A** to allow the motor unit **100A** to move the spool carriage **302** along the carriage track **301.**

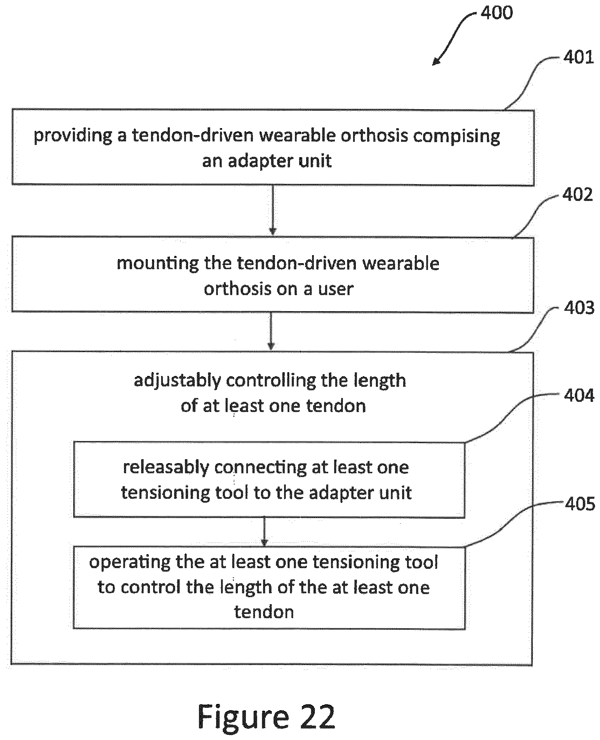

**Figure 22** shows a flow diagram of an exemplary method **400** for calibrating a tendon-driven wearable orthosis configured to be worn by a user.

In a first step **401,** the method **400** may comprise providing the tendon-driven wearable orthosis. The tendon-driven wearable orthosis may be configured to assist a movement of at least one joint of the user, and comprise an adapter unit connectable, via at least one tendon, to at least one end effector. The tendon-driven wearable orthosis may comprise any combination of features, as disclosed for a tendon-driven wearable orthosis herein.

In a second step **402,** the method **400** may further comprise mounting the tendon-driven wearable orthosis on the user. Mounting the tendon-driven wearable orthosis on the user may comprise, for example, at least partially wearing the tendon-driven wearable orthosis by the user.

In a third step **403,** the method **400** may further comprise adjustably controlling, by for example using at least one tensioning device of the adapter unit, a length of the at least one tendon between the adapter unit and the at least one end effector.

The third step **403** may further comprise a fourth step **404** of releasably connecting at least one tensioning tool to the adapter unit.

The third step **403** may further comprise a fifth step **405** of operating, such as for example by rotating and/or turning, the at least one tensioning tool to control the length of the at least one tendon between the adapter unit and the at least one end effector.

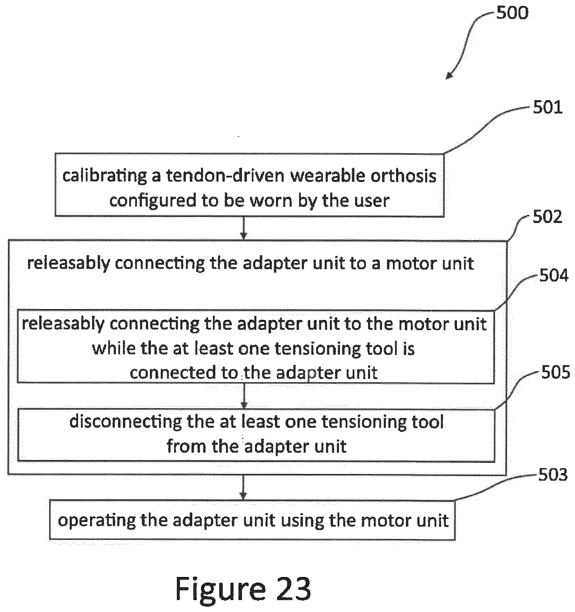

**Figure 23** shows a flow diagram of an exemplary method **500** for assisting a movement of at least one joint of a user. The method **500** may comprise, as a first step **501,** calibrating a tendon-driven wearable orthosis configured to be worn by the user. In particular, the first step **501** may comprise performing the method **400** for calibrating a tendon-driven wearable orthosis described herein.

In a second step **502,** the method **500** may comprise releasably connecting the adapter unit to a motor unit configured to operate the adapter unit to exert a pulling force on the at least one tendon to assist the movement of the at least one joint of the user. In a third step **503,** the method **500** may comprise operating the adapter unit using the motor unit.

The second step **502** may further comprise, in a fourth step **504,** releasably connecting the adapter unit to the motor unit while the at least one tensioning tool is connected to the adapter unit.

The second step **502** may in addition also comprise, in a fifth step **505,** disconnecting the at least one tensioning tool from the adapter unit.

As readily understood from the present disclosure, while the invention has been described by means of exemplary embodiments illustrated in the appended Figures, the invention is not limited thereto. Instead, any combination of features described herein and/or shown in the appended Figures may be implemented.

### List of Reference Numerals

- **1, 1A, 1B**: Adapter unit
- **10, 10A**: Adapter top plate
- **20, 20A**: Adapter bottom plate
- **11**: Top surface
- **12**: Top spool bore
- **13**: Second ratcheting teeth
- **14**: Tendon bore
- **15**: Top handling recess
- **16**: Bottom surface
- **17**: Top tendon groove
- **18**: Alignment protrusion
- **21**: Top surface
- **22**: Bottom spool bore
- **23**: Bottom tendon groove
- **24**: Groove protrusion
- **25**: Bottom handling recess
- **26**: Alignment recess
- **30**: First spool
- **31**: Central rotation shaft
- **32**: Tendon winding section
- **33**: First tensioning tool engagement section
- **34**: First motor unit engagement section
- **40**: Ratcheting key
- **41**: Handle
- **42**: Rotation direction indicator
- **43**: First ratcheting teeth
- **44**: First spool engagement section
- **50**: Spool bearing
- **60**: Tendon
- **70**: End effector
- **100,100A**: Motor unit
- **101**: Drive shaft
- **102**: Motor unit spool engagement section
- **103**: Adapter mounting surface
- **101A**: Bearing element
- **102A**: Motor unit housing
- **103A**: Spindle
- **104A**: Sled
- **105A**: Keyed connection post
- **106A**: Motor element
- **200, 200A**: Multi-spool unit
- **210, 210A**: Third spool
- **211**: Cylindrical central section
- **212**: Central mounting bore
- **213**: Locking bore
- **214**: Mounting plate
- **215**: Circumferential wall
- **216**: Opening
- **240**: Central rotation shaft
- **220**: Top plate
- **230**: Bottom plate
- **221**: Top plate motor engagement section
- **222**: Central top plate mounting bore
- **231**: Central bottom plate mounting bore
- **232**: Bottom plate locking bore
- **250, 250A**: Third tensioning tool
- **12A**: Multi-spool unit recess
- **22A**: Multi-spool accommodation space
- **26A**: Alignment post
- **27**: Access slit
- **28**: Tendon outlet
- **217**: Gripping protrusion
- **251**: Locking protrusion
- **252**: Access bore
- **300**: housing
- **301**: Carriage track
- **302**: Spool carriage
- **303**: Wheel element
- **304**: Second tensioning tool
- **305**: Fourth ratcheting teeth
- **306**: Third ratcheting teeth
- **307**: Keyed motor engagement section
- **308**: Spring element
- **309**: Second spool
- **R**: Rotation direction
- **400**: Method (for calibrating a tendon-driven wearable orthosis)
- **401-405**: Steps
- **500**: Method (for assisting a movement of at least one joint of a user)
- **501-505**: Steps

## Claims

1. A tendon-driven wearable orthosis configured to be worn by a user, wherein the tendon-driven wearable orthosis is configured to assist a movement of at least one joint of the user, comprising:
an adapter unit (1, 1A, 1B) connectable, via at least one tendon (60), to at least one end effector (70),
wherein the adapter unit (1, 1A, 1B) is releasably connectable to a motor unit (100, 100A), wherein the motor unit (100, 100A) is configured to operate the adapter unit (1, 1A, 1B) to exert a pulling force on the at least one tendon (60) to assist the movement of the at least one joint,
wherein the adapter unit (1, 1A, 1B) comprises at least one tensioning device configured to adjustably control a length of the at least one tendon (60) between the adapter unit (1, 1A, 1B) and the at least one end effector (70).

2. The tendon-driven wearable orthosis according to one of the preceding claims, wherein the tendon-driven wearable orthosis is configured to assist a movement of at least one finger joint of a hand of the user, and, optionally,
wherein the movement of at least one finger joint is a flexion and/or extension of the at least one finger joint.

3. The tendon-driven wearable orthosis according to one of the preceding claims, wherein the tendon-driven wearable orthosis further comprises the at least one tendon (60) and the at least one end effector (70); and/or
wherein the at least one tensioning device is configured to adjustably control the length of the at least one tendon (60) between the adapter unit (1, 1A, 1B) and the at least one end effector (70) while the adapter unit (1, 1A, 1B) is not connected to the motor unit (100, 100A).

4. The tendon-driven wearable orthosis according to one of the preceding claims, wherein the adapter unit (1, 1A, 1B) further comprises at least one first spool (30), wherein the at least one tendon (60) is connectable to the at least one first spool (30),
wherein the at least one first spool (30) is configured to be rotatable by the motor unit (100, 100A) to exert the pulling force on the at least one tendon (60).

5. The tendon-driven wearable orthosis according to claim 4, wherein the at least one tensioning device comprises at least one first tensioning tool releasably engageable with the adapter unit (1, 1A, 1B) and the at least one first spool (30),
wherein the at least one first spool (30) is configured to be operable using the at least one first tensioning tool to control the length of the at least one tendon (60) between the adapter unit (1, 1A, 1B) and the at least one end effector (70).

6. The tendon-driven wearable orthosis according to claim 5, wherein the at least one first tensioning tool is at least one ratcheting key (40),
wherein each ratcheting key (40) comprises a plurality of first ratcheting teeth (43) and the adapter unit (1, 1A, 1B) comprises a plurality of second ratcheting teeth (13),
wherein each ratcheting key (40) is configured to be releasably engageable with the adapter unit (1, 1A, 1B) and at least one first spool (30) to bring the plurality of first ratcheting teeth (43) into engagement with at least a subset of the plurality of second ratcheting teeth (13),
wherein the plurality of first ratcheting teeth (43) and at least the subset of plurality of second ratcheting teeth (13) are configured to prevent rotation of the at least one first spool (30) in a first rotation direction and allow rotation of the at least one first spool (30) in a second rotation direction opposite to the first rotation direction, while the plurality of first ratcheting teeth (43) is in engagement with at least the subset of plurality of second ratcheting teeth (13).

7. The tendon-driven wearable orthosis according to one of claims 1 to 3, wherein the at least one tensioning device comprises:
a carriage movably mounted on a carriage track (301),
at least one connection device mounted on the carriage, wherein the at least one tendon (60) is connectable to the at (east one connection device,
wherein the carriage is configured to be movable along the carriage track (301) by the motor unit (100, 100A) to exert the pulling force on the at least one tendon (60).

8. The tendon-driven wearable orthosis according to claim 7, wherein the carriage is a spool carriage (302),
wherein the at least one connection device is at least one second spool (309).

9. The tendon-driven wearable orthosis according to claim 8, wherein the spool carriage (302) comprises a plurality of third ratcheting teeth (306),
wherein the at least one second spool (309) comprises a plurality of fourth ratcheting teeth (305) releasably engageable with at least a subset of the plurality of third ratcheting teeth (306),
wherein the plurality of fourth ratcheting teeth (305) and at least the subset of plurality of third ratcheting teeth (306) are configured to prevent rotation of the at least one second spool (309) in a third rotation direction and allow rotation of the at least one second spool (309) in a fourth rotation direction opposite to the third rotation direction, while the plurality of fourth ratcheting teeth (305) is in engagement with at least the subset of plurality of third ratcheting teeth (306).

10. The tendon-driven wearable orthosis according to claim 9, wherein the at least one tensioning device comprises at least one second tensioning tool (304) releasably engageable with the at least one second spool (309),
wherein the at least one second spool (309) is configured to be rotatable in the fourth direction using the at least one second tensioning tool (304) to control the length of the at least one tendon (60) between the adapter unit (1, 1A, 1B) and the at least one end effector (70),
wherein the at least one second spool (309) is configured to be operable using the at least one second tensioning tool (304) to move the plurality of fourth ratcheting teeth (305) out of engagement with at least the subset of the plurality of third ratcheting teeth (306).

11. The tendon-driven wearable orthosis according to one of claims 1 to 3, wherein the at least one tensioning device further comprises a multi-spool unit (200, 200A), wherein the multi-spool unit (200, 200A) comprises:
a central rotation shaft (240),
a plurality of third spools (210, 210A) mounted on the central rotation shaft (240), wherein the at least one tendon (60) is connectable to the plurality of third spools (210, 210A),
wherein the multi-spool unit (200, 200A) is configured to be switchable between a first state and a second state,
wherein, in the first state, each of the plurality of third spools (210, 210A) is configured to be rotatable around the central rotation shaft (240),
wherein, in the second state, the plurality of third spools (210, 210A) is configured to be fixed relative to the central rotation shaft (240),
wherein, in the second state, the multi-spool unit (200, 200A) is configured to be rotatable by the motor unit (100, 100A) to exert the pulling force on the at least one tendon (60).

12. The tendon-driven wearable orthosis according to claim 11, wherein the at least one tensioning device comprises at least one third tensioning tool (250, 250A),
wherein the at least one third tensioning tool (250, 250A) is configured to be releasably engageable with the plurality of third spools (210, 210A) in the first state to fix an orientation of the plurality of third spools (210, 210A) relative to one another.

13. The tendon-driven wearable orthosis according to one claims 5, 6, 10, or 12,
wherein the adapter unit (1, 1A, 1B) is configured to be connectable to the motor unit (100, 100A) and the at least one first tensioning tool, the at least one second tensioning tool (304), or the at least one third tensioning tool (250, 250A), respectively, at the same time.

14. A method (400) for calibrating a tendon-driven wearable orthosis configured to be worn by a user, wherein the tendon-driven wearable orthosis is configured to assist a movement of at least one joint of the user, wherein the tendon-driven wearable orthosis comprises an adapter unit (1, 1A, 1B) connectable, via at least one tendon (60), to at least one end effector (70), the method comprising:
mounting the tendon-driven wearable orthosis on the user;
adjustably controlling, using at least one tensioning device of the adapter unit (1, 1A, 1B), a length of the at least one tendon (60) between the adapter unit (1, 1A, 1B) and the at least one end effector (70).

15. The method (400) for calibrating a tendon-driven wearable robot according to claim 14, wherein the adjustably controlling a length of the at least one tendon (60) comprises:
releasably connecting at least one tensioning tool to the adapter unit (1, 1A, 1B),
operating, using the at least one tensioning tool, the at least one tensioning device to control the length of the at least one tendon (60) between the adapter unit (1, 1A, 1B) and the at least one end effector (70).
